(19) Europäisches Patentamt
European Patent Office
Office européen des brevets

(11) Veröffentlichungsnummer: **0 098 486**
**B1**

(12) **EUROPÄISCHE PATENTSCHRIFT**

(45) Veröffentlichungstag der Patentschrift: **03.09.86**

(21) Anmeldenummer: **83106205.4**

(22) Anmeldetag: **25.06.83**

(51) Int. Cl.⁴: **C 07 D 401/12**, C 07 D 409/12, C 07 D 413/12, C 07 D 417/12, A 01 N 43/42

(54) **Chinolinderivate, Verfahren zu ihrer Herstellung, diese enthaltende Mikrobizide und ihre Verwendung zur Bekämpfung von Pilzen.**

(30) Priorität: **06.07.82 DE 3225169**

(43) Veröffentlichungstag der Anmeldung: **18.01.84 Patentblatt 84/3**

(45) Bekanntmachung des Hinweises auf die Patenterteilung: **03.09.86 Patentblatt 86/36**

(84) Benannte Vertragsstaaten: **AT BE CH DE FR GB IT LI NL SE**

(56) Entgegenhaltungen:
**EP-A-0 006 506**
**DE-A-2 105 174**
**DE-A-2 359 773**
**FR-A-1 503 697**
**GB-A-1 420 768**
**US-A-4 277 479**

(73) Patentinhaber: **BASF Aktiengesellschaft, Carl-Bosch- Strasse 38, D-6700 Ludwigshafen (DE)**

(72) Erfinder: **Hamprecht, Gerhard, Dr., Rote- Turm-Strasse 28, D-6940 Weinheim (DE)**
Erfinder: **Markert, Juergen, Dr., Am Speyerweg 26, D-6704 Mutterstadt (DE)**
Erfinder: **Spiegler, Wolfgang, Dr., Amsterdamer Strasse 16, D-6700 Ludwigshafen (DE)**
Erfinder: **Richarz, Winfried, Dr., Königsberger Strasse 5, D-6081 Stockstadt (DE)**
Erfinder: **Graf, Hermann, Dr., Ginsterstrasse 15, D-6704 Mutterstadt (DE)**
Erfinder: **Ammermann, Eberhard, Dr., Sachsenstrasse 3, D-6700 Ludwigshafen (DE)**
Erfinder: **Pommer, Ernst- Heinrich, Dr., Berliner Platz 7, D-6703 Limburgerhof (DE)**

**EP 0 098 486 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft neue Chinolinderivate, Verfahren zu ihrer Herstellung, Mikrobizide, die diese Verbindungen als Wirkstoffe enthalten, sowie Verfahren zur Bekämpfung von Pilzen.

Es ist bekannt, den 5-Chlor-7-brom-8-hydroxychinolinacrylsäureester zur Bekämpfung von Pilzen zu verwenden (DE-21 05 174). Er zeigt jedoch nur unzureichende Wirkung gegen Botrytis.

Es wurde nun gefunden, daß substituierte Chinolinderivate der Formel

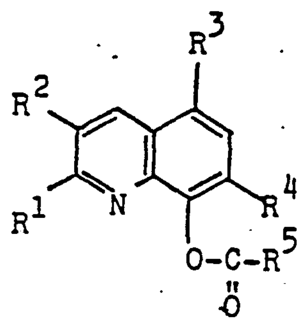

I

in der

$R^1$ Wasserstoff oder Methyl,

$R^2$ Wasserstoff oder Halogen,

$R^3$ Wasserstoff, Acetyl, Halogen oder Nitro

$R^4$ Wasserstoff, Halogen oder Nitro und

$R^5$ einen gegebenenfalls durch einen $C_1$-$C_4$-Alkylrest, einen $C_1$-$C_4$-Halogenalkylrest, eine Nitrogruppe oder ein Halogenatom einfach oder unabhängig voneinander zweifach substituierten Thiophen, Pyrrol-, Oxazol-, Thiazol-, Imidazol-, Isoxazol-, Isothiazol-, Pyrazol-, 1,3,4-Thiadiazol-, 1,3,4-Oxdiazol-, 1,2,4-Thiadiazol-, 1,2,4-Oxdiazol-, 1,2,4-Triazol-, 1,2,3-Thiadiazol-, 1,2,3-Oxadiazol-, 1,2,3-Triazol-, 1,2,5-Thiadiazol- oder 1,2,5-Oxadiazol-Rest oder einen halogen- oder mono- oder dimethylsubstituierten Furan-Rest bedeuten,

mikrobizid ausgezeichnet wirksam sind.

$R^5$ bedeutet beispielsweise einen gegebenenfalls durch einen $C_1$-$C_4$-Alkylrest, einen $C_1$-$C_4$-Halogenalkylrest, eine Nitrogruppe oder ein Halogenatom einfach oder unabhängig voneinander zweifach substituierten Thiophen-(2)-, -(3)-, -(4)- oder -(5-)-Rest, Pyrrol-(2)-, -(3)-, -(4-)- oder -(5-)-Rest, Oxazol-(2-)-, -(4-)- oder -(5-)-Rest, Thiazol-(2)-, -(4-)- oder -(5)-Rest, Imidazol-(2)-, -(4)- oder -(5)-Rest, Isoxazol-(3)-, -(4)- oder -(5)-Rest, Isothiazol-(3)-, -(4)- oder -(5)-Rest, Pyrazol-(3)-, -(4)-oder -(5)-Rest, 1,3,4-Thiadiazol-(2)- oder -(5)-Rest, 1,3,4-Oxadiazol-(2)- oder -(5)-Rest, 1,2,4-Thiadiazol-(3)-oder -(5)-Rest, 1,2,4-Oxadiazol-(3)- oder -(5)-Rest, 1,2,4-Triazol-(3)- oder -(5)-Rest, 1,2,3-Thiadiazol-(4)- oder -(5-)-Rest, 1,2,3-Oxadiazol-(4)- oder -(5)-Rest, 1,2,3-Triazol-(4)- oder -(5)-Rest, 1,2,5-Thiadiazol-(3)- oder -(4)-Rest oder 1,2,5-Oxadiazol-(3)- oder -(4)-Rest, oder einen Halogen- oder mono- oder dimethylsubstituierten Furan-(2)-, -(3)-, -(4)- oder -(5-Rest). Halogen bedeutet beispielsweise Chlor oder Brom.

Bevorzugt werden Verbindungen der Formel I, in der $R^1$ Wasserstoff, $R^2$ Wasserstoff oder Halogen, $R^3$ Acetyl, Halogen oder Nitro, $R^4$ Halogen oder Nitro und $R^5$ einen gegebenenfalls durch Chlormethyl substituierten Isoxsazol-(3)- oder -(5)-Rest, einen gegebenenfalls durch Methyl oder Chlor substituierten Thiophen-(2)-, -(3)- oder -(5-)-Rest, einen gegebenenfalls durch Methyl substituierten Oxazol-(5)-Rest, oder einen gegebenenfalls durch Methyl substituierten 1,2,3-Thiadiazol-(4)- oder -(5)-Rest bedeutet.

Die Herstellung der Verbindungen der Formel I erfolgt beispielsweise dadurch daß man eine Verbindung der Formel II

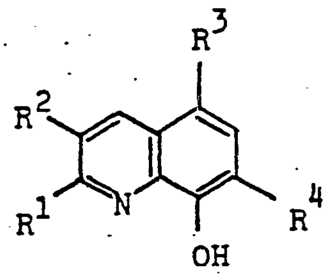

II

in der
$R^1$, $R^2$, $R^3$ und $R^4$ die angegebene Bedeutung haben oder ihre Alkali- oder Erdalkalisalze mit einem Carbonsäurederivat der Formel III

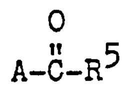

III

in der
$R^5$ die oben angegebene Bedeutung hat und A für eine nucleophil verdrängbare Abgangsgruppe steht, umsetzt.

In der Formel III bedeutet A beispielsweise Halogen, wie Chlor oder Brom, einen Alkoxycarbonyloxyrest, wie Methoxycarbonyloxy und Ethoxycarbonyloxy, den Benzyloxycarbonyloxyrest, einen Azolylrest, wie den Imidazolyl- oder den Triazolylrest oder einen Alkoxyrest wie den Methoxy- oder Ethoxyrest.

Obwohl die Reaktion auch in Abwesenheit von Lösungsmitteln durchgeführt werden kann, ist es zweckmäßig, die Umsetzung in einem inerten Lösungs- oder Verdünnungsmittel vorzunehmen. Als Lösungsmittel kommen z.B. in Frage. Halogenkohlenwasserstoffe, insbesondere Chlorkohlenwasserstoffe, z.B. Tetrachlorethylen, 1,1,2,2- der 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, Iodbenzol, o-, m-, p-Dichlorbenzol, o-, p-, m-Dibrombenzol, o-, m-, p-Chlortoluol, 1,2,4-Trichlorbenzol; Ether, z.B. Ethylpropylether, Methyl-tert.-butylether, n-Butylethylether, Di-n-butylether, Diisobutylether, Diisoamylether, Diisopropylether, Anisol, Phenetol, Cyclohexylmethylether, Diethylether, Ethylenglykoldimethylether, Tetrahydrofuran, Dioxan, Thioanisol, β, β'-Dichlordiethylether; Nitrokohlenwasserstoffe, wie Nitromethan, Nitroethan, Nitrobenzol, o-, m-, p-Chlornitrobenzol, o-Nitrotoluol; Nitrile wie Acetonitril, Butyronitril, Isobutyronitril, Benzonitril, m-Chlorbenzonitril; aliphatische oder cycloaliphatische Kohlenwasserstoffe, z.B. Heptan, Pinan, Nonan, o-, m-, p-Cymol, Benzinfraktionen innerhalb eines Siedepunktintervalles von 70 bis 190°C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan; Ester, z.B. Ethylacetat, Acetessigester, Isobutylacetat; Amide, z.B. Formamid, Methylformamid, Dimethylformamid; Ketone, z.B. Aceton, Methylethylketon, gegebenenfalls auch Wasser und entsprechende Gemische. Zweckmäßig verwendet man das Lösungsmittel in einer Menge von 100 bis 2000 Gew.%, vorzugsweise von 200 bis 900 Gew.%., bezogen auf Ausgangsstoff II.

Es empfiehlt sich - obwohl es nicht notwendig ist -, die Umsetzung in Gegenwart eines Säureakzeptors durchzuführen. Als solche können alle üblichen Säurebindemittel verwendet werden. Hierzu gehören vorzugsweise tertiäre Amine, Erdalkaliverbindungen, Ammoniumverbindungen und Alkaliverbindungen sowie entsprechende Gemische. Es können aber auch Zinkverbindungen verwendet werden. Als basische Verbindungen kommen z.B. in Frage: Kaliumhydroxid, Natriumhydroxid, Kaliumcarbonat, Natriumcarbonat, Lithiumhydroxid, Lithiumcarbonat, Natriumhydrogencarbonat, Kaliumhydrogencarbonat, Calciumhydroxid, Bariumoxid, Magnesiumhydroxid, Magnesiumoxid, Bariumhydroxid, Calciumcarbonat, Magnesiumcarbonat, Magnesiumacetat, Zinkhydroxid, Zinkoxid, Zinkcarbonat, Zinkacetat, Natriumformiat, Natriumacetat, Trimethylamin, Triethylamin, Tripropylamin, Triisopropylamin, Tributylamin, Triisobutylamin, Tri-sec-butylamin, Tri-tert.-butylamin, Tribenzylamin, Tricyclohexylamin, Triamylamin, Trihexylamin, N,N-Dimethylanilin, N,N-Diethylanilin, N,N-Dipropylanilin, N,N-Dimethyltoluidin, N,N-Diethyltoluidin, N,N-Dipropyltoluidin, N,N-Dimethyl-p-aminopyridin, N,N-Diethyl-p-aminopyridin, N,N-Dipropyl-p-aminopyridin, N-Methylpyrrolidin, N-Ethylpyrrolidon, N-Methylpiperidin, N-Ethylpiperidin, N-Methyl-pyrrolidin, N-Ethylpyrrolidin, N-Methylimidazol, N-Ethylimidazol, N-Methylpyrrol, N-Ethylpyrrol, N-Methylmorpholin, N-Ethylmorpholin, N-Methylhexamethylenimin, N-Ethylhexamethylenimin, Pyridin, Chinolin, alpha-Picolin, β-Picolin, gamma-Picolin,

Isochinolin, Pyrimidin, Acridin, N,N,N',N'-Tetramethylethylendiamin, N,N,N',N'-Tetraethylethylendiamin, Chinoxalin, Chinazolin, N-Propyldiisopropylamin, N,N-Dimethylcyclohexylamin, 2,6-Lutidin, 2,4-Lutidin, Trifurfurylamin, Triethylendiamin.

Man kann jedoch auch den bei der Reaktion beispielsweise entstehenden Halogenwasserstoff durch Einleiten eines Inertgases, beispielsweise Stickstoff, entfernen.

Zweckmäßig wird das Verfahren zur Herstellung der neuen Verbindungen so durchgeführt, daß man den Ausgangsstoff II, gegebenenfalls in einem der vorgenannten Verdünnungsmittel vorlegt und dann den Ausgangsstoff III und einen Säureakzeptor gleichzeitig oder nacheinander zugibt. Man kann jedoch auch den Ausgangsstoff III in einem der vorgenannten Verdünnungsmittel vorlegen und dann den Ausgangsstoff II und einen Säureakzeptor, gleichzeitig oder in beliebiger Reihenfolge, über zwei getrennte Zuführungen zugeben.

Die Umsetzung ist in vielen Fällen nach der Zugabe der Komponenten bereits abgeschlossen, andernfalls rührt man zu ihrer Beendigung noch 10 Minuten bis 10 Stunden bei -10 bis 120°C, vorzugsweise 0 bis 100°C, insbesondere 20 bis 80°C nach.

Aus dem Reaktionsgemisch wird die Verbindung I in üblicher Weise, z.B. durch Abdestillieren von Lösungsmittel oder überschüssigem Ausgangsstoff III oder direkt durch Absaugen isoliert. Der verbleibende Rückstand wird in diesem Fall zur Entfernung saurer oder basischer Verunreinigungen mit Wasser bzw. verdünntem Alkali oder Säure gewaschen und getrocknet. Im Fall von mit Wasser nicht mischbaren Verdünnungsmitteln kann man auch direkt das Reaktionsgemisch mit Wasser bzw. mit verdünntem Alkali oder Säure extrahieren und dann trocknen und einengen. Man kann jedoch auch den Rückstand in einem mit Wasser nicht mischbaren Lösungsmittel lösen und wie beschrieben waschen. Die gewünschten Endstoffe fallen hierbei in reiner Form an, gegebenenfalls können sie durch Umkristallisation, Chromatographie oder Destillation weiter gereinigt werden.

Die nachfolgenden Beispiele dienen zur näheren Erläuterung der Erfindung, ohne dieselbe einzuschränken.

**Beispiel 1**

10,2 Teile (Gew.-Teile) Triethylamin wurden bei 20 bis 25°C unter Rühren zu einer Mischung von 25,9 Teilen 5-Chlor-7-brom-8-hydroxy-chinolin in 170 Teilen Essigester gegeben. Anschließend wurden 16,3 Teile 5-Methyl-1,2,3-thiadiazol-4-carbonsäurechlorid in 30 Teilen Essigester unter Rühren bei 20 bis 25°C zugetropft. Nach 1 1/2 Stunden Rühren bei 25°C wurde der ausgefallene Niederschlag abgesaugt, in 100 Teilen Wasser unter Zugabe von 5 Teilen 1 normale Salzsäure kurz aufgerührt und wieder abgesaugt. Zum Trocknen wurde der Rückstand in Methylenchlorid aufgenommen, mit Magnesiumsulfat behandelt und filtriert. Nach dem Destillieren des Lösungsmittels im Vakuum wurde der verbleibende Rückstand mit Methyl-tert.-butylether verrieben, abgesaugt und getrocknet, wobei 32,5 Teile 5-Chlor-7-brom-8-(5'-methyl-1',2',3'-thiadiazol-4'-carbonyl-)-oxychinolin vom Fp 178 bis 180°C erhalten wurden (Verbindung Nr. 1).

**Beispiel 2**

12,6 Teile Dimethylcyclohexylamin und 14,9 Teile 1,2,3-Thiadiazol-4-carbonsäurechlorid in 20 Teilen Methylenchlorid wurden parallel über 2 Zuführungen unter Rühren zu einer Mischung von 18 Teilen 5-Chlor-8-hydroxychinolin in 150 Teilen Methylenchlorid zugegeben, wobei sich die Reaktionsmischung bis auf 40°C erwärmt. Nach dem Abkühlen auf Raumtemperatur unter Rühren innerhalb von 15 Minuten wurden 150 Teile Wasser und 5 Teile 1 normale Salzsäure zugegeben und die Phasen getrennt. Die organische Phase wurde nochmals mit Wasser gewaschen, getrocknet und im Vakuum eingeengt. Der Rückstand wurde mit Methyl-tert.-butylether verrührt, abgesaugt und getrocknet, wobei 24 Teile 5-Chlor-8-(1',2',3'-thiadiazolyl-4'-carbonyl-)--oxychinolin vom Fp. 160 bis 163°C erhalten wurden (Verbindung Nr. 2).

**Beispiel 3**

25,9 Teile 5-Chlor-7-brom-8-hydroxychinolin in 120 Teilen Diethylether und 7,9 Teile Pyridin wurden bei 0°C zu 16,1 Teilen 2-Methyl-thiophen-5-carbonsäurechlorid in 100 Teilen Diethylether unter Rühren innerhalb 10 Minuten gegeben. Das Reaktionsgemisch wurde auf 35°C erwärmt und 15 Minuten nachgerührt. Nach dem Absaugen des ausgefallenen Niederschlages wurde das Filtrat einmal mit Wasser, zweimal mit 0,5 normaler Salzsäure extrahiert und eingeengt. Der Rückstand wurde mit dem auf gleiche Weise wie oben mit Wasser und Salzsäure gewaschenen Niederschlag vereinigt und in Aceton aufgerührt, wobei 30,5 Teile 5-Chlor-7-brom-8-(2'-methyl-thiophen-5'-carbonyl-)-oxychinolin vom Fp. 143 bis 146°C erhalten wurden (Verbindung Nr. 3).

Entsprechend wurden diejenigen in der folgenden Tabelle angegebenen Verbindungen der Formel I hergestellt, deren Schmelzpunkte (Fp) angegeben sind. Ihre Struktur wurde durch Elementaranalyse gesichert. Die Verbindungen, bei denen keine Angaben zur physikalischen Chemie gemacht sind, können auf die gleiche

Art und Weise wie bei den tatsächlich hergestellten Verbindungen erhalten werden; es kann erwartet werden, daß sie aufgrund ihrer ähnlichen Konstitution ähnliche Wirkungen wie die näher untersuchten Verbindungen haben.

| Nr. | $R^1$ | $R^2$ | $R^3$ | $R^4$ | $R^5$ | Fp ($^oC$) |
|---|---|---|---|---|---|---|
| 4 | H | H | Cl | Br | Thienyl-2 | 118-120 |
| 5 | H | H | Cl | Br | Thienyl-3 | 159-162 |
| 6 | H | H | Cl | Cl | 2-Chlor-thienyl-3 | |
| 7 | H | H | Cl | Br | 5-Chlor-thienyl-2 | 117-120 |
| 8 | H | H | Cl | Br | 5-Nitro-thienyl-2 | |
| 9 | H | H | Cl | Br | 4-Methylthienyl-2 | |
| 10 | H | H | Cl | Br | 3-Methyl-thienyl-2 | 149-153 |
| 11 | H | H | Cl | Br | 2-Methyl-thienyl-3 | |
| 12 | H | H | Cl | Br | 3-Methyl-thienyl-4 | |
| 13 | H | H | Cl | Br | 2,5-Dimethyl-thienyl-3 | |
| 14 | H | H | Cl | Br | 2,3-Dichlor-thienyl-4 | |
| 15 | H | H | Br | Br | 5-Methyl-thienyl-2 | |
| 16 | H | H | Cl | Br | Pyrryl-2 | |
| 17 | H | H | Cl | Br | Pyrryl-3 | |
| 18 | H | H | Cl | Br | 3-Methyl-pyrryl-2 | |
| 19 | H | H | Cl | Br | 2-Methyl-pyrryl-3 | |
| 20 | H | H | Cl | Br | 5-Chlor-pyrryl-2 | |
| 21 | H | H | Cl | Br | Oxazolyl-2 | |
| 22 | H | H | Cl | Br | Oxazolyl-4 | |
| 23 | H | H | Cl | Br | Oxazolyl-5 | |
| 24 | H | H | $CH_3-C=O$ | $NO_2$ | 1,2,3-Thiadiazolyl-4 | 165 Zersetzung |

| Nr. | $R^1$ | $R^2$ | $R^3$ | $R^4$ | $R^5$ | Fp ($^oC$) |
|---|---|---|---|---|---|---|
| 25 | H | H | $CH_3-C=O$ | Cl | 1,2,3-Thiadiazolyl-4 | |
| 26 | H | H | Cl | Br | 4-Methyl-oxazolyl-5 | 129-133 |
| 27 | H | H | Cl | Br | 2-Methyl-oxazolyl-5 | |
| 28 | H | H | Cl | Br | Thiazolyl-2 | |
| 29 | H | H | Cl | Br | Thiazolyl-4 | |
| 30 | H | H | Cl | Br | Thiazolyl-5 | |
| 31 | H | H | Cl | Br | 4-Methyl-thiazolyl-5 | |
| 32 | H | H | Cl | Br | 2-Methyl-thiazolyl-5 | |
| 33 | H | H | Cl | Br | Imidazolyl-4 | |
| 34 | H | H | Cl | Br | Imidazolyl-5 | |
| 35 | H | H | Cl | Br | 4-Methyl-imidazolyl-5 | |
| 36 | H | H | Cl | Br | 1-Methyl-imidazolyl-5 | |
| 37 | H | H | Cl | Br | 1-Methyl-pyrryl-2 | |
| 38 | H | H | Cl | Br | Isoxazolyl-3 | 131-134 |
| 39 | H | H | $CH_3-C=O$ | $NO_2$ | Isoxazolyl-3 | |
| | | H | | | | |
| 40 | H | H | $CH_3-C=O$ | Cl | Isoxazolyl-3 | |
| | | H | | | | |
| 41 | $CH_3$ | H | Br | Br | Isoxazolyl-3 | |
| 42 | H | H | Cl | Br | Isoxazolyl-4 | |
| 43 | H | H | Cl | Br | Isoxazolyl-5 | 128-130 |

| Nr. | $R^1$ | $R^2$ | $R^3$ | $R^4$ | $R^5$ | Fp (°C) |
|---|---|---|---|---|---|---|
| 44 | H | H | J | J | Isoxazolyl-5 | |
| 45 | H | H | Br | Br | Isoxazolyl-5 | |
| 46 | H | H | Cl | Cl | Isoxazolyl-5 | |
| 47 | $CH_3$ | H | H | H | Isoxazolyl-5 | |
| 48 | H | H | Cl | Br | 5-Chlormethyl-isoxazolyl-3 | 108-112 |
| 49 | H | H | Cl | Br | Isothiazolyl-3 | |
| 50 | H | H | Cl | Br | Isothiazolyl-4 | 158-160 |
| 51 | H | H | Cl | Br | Isothiazolyl-5 | |
| 52 | H | H | Cl | Br | 4-Methyl-isothiazolyl-5 | |
| 53 | H | H | Cl | Br | Pyrazolyl-4 | |
| 54 | H | H | Cl | Br | Pyrazolyl-5 | |
| 55 | H | H | Cl | Br | 4-Chlor-pyrazolyl-5 | |
| 56 | H | H | Cl | Br | 1-Methyl-pyrazolyl-5 | |
| 57 | H | H | Cl | Br | 1,2,3-Thiadiazolyl-4 | 176-180 |
| 58 | H | H | Cl | Cl | 1,2,3-Thiadiazolyl-4 | 170-172 |
| 59 | H | H | J | J | 1,2,3-Thiadiazolyl-4 | 175-179 |
| 60 | H | H | Br | Br | 1,2,3-Thiadiazolyl-4 | 175-178 |
| 61 | H | H | Cl | Br | 1,2,3-Oxadiazolyl-4 | |
| 62 | H | H | Cl | Br | 1,2,3-Oxadiazolyl-5 | |
| 63 | H | H | Cl | Br | 5-Methyl-1,2,3-oxadiazolyl-4 | |
| 64 | H | H | Cl | J | 1,2,3-Thiadiazolyl-4 | 140-143 |
| 65 | $CH_3$ | H | Br | Br | 1,2,3-Thiadiazolyl-4 | 175-178 |

| Nr. | $R^1$ | $R^2$ | $R^3$ | $R^4$ | $R^5$ | Fp (°C) |
|---|---|---|---|---|---|---|
| 66 | $CH_3$ | H | H | H | 1,2,3-Thiadiazolyl-4 | 121-124 |
| 67 | H | H | Cl | Br | 1,2,3-Thiadiazolyl-5 | 126-134 |
| 68 | H | H | Cl | Br | 4-Methyl-1,2,3-oxadiazolyl-5 | |
| 69 | H | H | Cl | Br | 1,3,4-Thiadiazolyl-2 | |
| 70 | H | H | Cl | Br | 2-Methyl-1,3,4-thiadiazolyl-5 | |
| 71 | H | H | Cl | Br | 1,2,4-Thiadiazolyl-3 | |
| 72 | H | H | Cl | Br | 1,2,4-Thiadiazolyl-5 | |
| 73 | H | H | Cl | Br | 3-Methyl-1,2,4-thiadiazolyl-5 | |
| 74 | H | H | Cl | Br | 1,2,4-Oxadiazolyl-3 | |
| 75 | H | H | Cl | Br | 1,2,4-Oxadiazolyl-5 | |
| 76 | H | H | Cl | Br | 1,2,4-Triazolyl-5 | |
| 77 | H | H | Cl | Br | 1,2,4-Triazolyl-3 | |
| 78 | H | H | $NO_2$ | H | 1,2,3-Thiadiazolyl-4 | 172-175 Zersetzung |
| 79 | H | H | Cl | Br | 1,3,4-Oxadiazolyl-5 | |
| 80 | H | H | Cl | Br | 2-Methyl-1,3,4-oxadiazolyl-5 | |
| 81 | H | H | Cl | Br | 2-Chlor-1,3,4-oxadiazolyl-5 | |
| 82 | H | H | Cl | Br | 1,2,5-oxadiazolyl-3 | |
| 83 | H | H | Cl | Br | 3-Methyl-1,2,5-oxadiazolyl-4 | |
| 84 | H | H | Cl | Br | 1,2,5-Thiadiazolyl-3 | |
| 85 | H | H | Cl | Br | 3-Methyl-1,2,5-thiadiazolyl-4 | |
| 86 | H | H | Cl | Cl | 2-Methyl-furoyl-4 | |

| Nr. | $R^1$ | $R^2$ | $R^3$ | $R^4$ | $R^5$ | Fp (°C) |
|---|---|---|---|---|---|---|
| 87 | H | H | Cl | Cl | 2-Methyl-furoyl-5 | |
| 88 | H | H | Cl | Br | 2,5-Dimethyl-furoyl-4 | 104-106 |
| 89 | H | H | Cl | Br | 2-Chlor-furoyl-5 | |
| 90 | H | H | Cl | Br | 5-Methyl-oxazolyl-4 | |
| 91 | H | H | Cl | Br | 4-Methyl-oxazolyl-2 | |
| 92 | H | H | Cl | Br | 2-Brom-furoyl-5- | 150-152 |
| 93 | H | H | Cl | Br | 2-Methyl-furoyl-5 | 141-143 |
| 94 | H | H | Cl | Br | 5-Methyl-isoxazolyl-3 | |
| 95 | H | H | Cl | Br | 4-Methyl-isoxazolyl-3 | |
| 96 | H | H | Cl | Br | 5-Methyl-isothiazol-3 | |
| 97 | H | H | Cl | Br | 4-Methyl-isothiazol-3 | |
| 98 | H | Cl | Cl | Br | 5-Methyl-oxazolyl-4 | |
| 99 | H | Cl | Cl | H | 1,2,3-Thiadiazolyl-4 | |
| 100 | H | Cl | Cl | H | 1,2,3-Thiadiazolyl-5 | |
| 101 | H | Cl | Cl | H | Isoxazolyl-3 | |
| 102 | H | Cl | Cl | H | Isoxazolyl-5 | |
| 103 | H | Cl | Cl | H | Isoxazolyl-4 | |
| 104 | H | Cl | Cl | H | 5-Methyl-isoxazolyl-3 | |
| 105 | H | Cl | Cl | H | 5-Chlormethyl-isoxazolyl-3 | |
| 106 | H | Cl | Cl | Cl | 1,2,3-Thiadiazolyl-4 | 176-178 |
| 107 | H | Cl | Cl | Cl | 1,2,3-Thiadiazolyl-5 | |
| 108 | H | Cl | Cl | Cl | Isoxazolyl-3 | |

| Nr. | $R^1$ | $R^2$ | $R^3$ | $R^4$ | $R^5$ | Fp (°C) |
|---|---|---|---|---|---|---|
| 109 | H | Cl | Cl | Cl | Isoxazolyl-4 | |
| 110 | H | Cl | Cl | Cl | Isoxazolyl-5 | |
| 111 | H | Cl | Cl | Cl | 5-Methyl-isoxazolyl-3 | |
| 112 | H | Cl | Cl | Cl | 5-Chlormethyl-isoxazolyl-3 | |
| 113 | H | Cl | H | Cl | 1,2,3-Thiadiazolyl-4 | 144-147 |
| 114 | H | Cl | H | Cl | 5-Methyl-1,2,3-thiadiazolyl-4 | |
| 115 | H | Cl | H | Cl | Isoxazolyl-3 | |
| 116 | H | Cl | H | Cl | Isoxazolyl-5 | |
| 117 | H | Cl | H | Cl | 5-Chlormethyl-isoxazolyl-3 | |
| 118 | H | Cl | Cl | Br | 1,2,3-Thiadiazolyl-4 | |
| 119 | H | Cl | Cl | Br | 1,2,3-Thiadiazolyl-5 | |
| 120 | H | Cl | Cl | Br | Isoxazolyl-3 | |
| 121 | H | Cl | Cl | Br | Isoxazolyl-5 | |
| 122 | H | Cl | Cl | Br | 5-Chlormethyl-isoxazolyl-3 | |
| 123 | H | Br | Br | Cl | 1,2,3-Thiadiazolyl-4 | |
| 124 | H | H | Cl | Br | 1,2,3-Thiazolyl-4 | |
| 125 | H | H | Cl | Br | 1,2,4-Thiazolyl-1 | |
| 126 | H | H | Cl | Br | 3-Methyl-isothiazolyl-4 | |
| 127 | H | Cl | Cl | Cl | 4-Methyl-oxazolyl-5 | |
| 128 | H | H | Cl | H | 4-Methyl-1,2,3-thiadiazolyl-5 | 170-172 |
| 129 | H | H | Cl | Br | 4-Methyl-1,2,3-thiadiazolyl-5 | 121-123 |
| 130 | H | H | Cl | Cl | 4-Methyl-1,2,3-thiadiazolyl-5 | 122-123 |
| 131 | H | Cl | Br | Cl | 1,2,3-Thiadiazolyl-4 | 149-152 |

Die Wirkstoffe zeigen eine starke Wirksamkeit gegen Mikroorganismen. Sie dienen insbesondere zur Verhütung und Heilung von Pflanzenkrankheiten, die durch Pilze verursacht werden, wie z.B. Botrytis cinerea an Reben und Erdbeeren, Monilia fructigena an Äpfeln, Phytophthora infestans an Kartoffeln und Tomaten, Plasmopara viticola an Reben, Alternaria solani an Tomaten und Helminthosporium an Getreide. Ferner zeigen sie eine gute Wirksamkeit im Materialschutz gegen holzverfärbende und holzzerstörende Pilze wie Chaetomium globosum, Pullularia pullulans, Sclerophoma pityophyla, Aspergillus niger, Coniophora puteana und Polystictus versicolor. Außerdem besitzen die Wirkstoffe eine vorteilhafte bakterizide Wirksamkeit zum Beispiel gegen Staphylococcus aureus, Escherichia coli, Xanthomonas- und Pseudomonas-Arten.

Die fungiziden bzw. bakteriziden Mittel enthalten 0,1 bis 95 % (Gew.%) Wirkstoff, vorzugsweise 0,5 bis 90 %. Die Aufwandmengen liegen je nach Art des gewünschten Effektes zwischen 0,1 und 5 kg Wirkstoff je ha.

Die Wirkstoffe können auch zusammen mit anderen Wirkstoffen, z.B. Herbiziden, Insektiziden, Wachstumsregulatoren und anderen Fungiziden, oder auch mit Düngemitteln vermischt und ausgebracht werden. In vielen Fällen erhält man bei der Mischung mit Fungiziden auch eine Vergrößerung des fungiziden Wirkungsspektrums; bei einer Anzahl dieser Fungizidmischungen treten auch synergistische Effekte auf, d.h. die fungizide Wirksamkeit des Kombinationsproduktes ist größer als die der addierten Wirksamkeiten der Einzelkomponenten. Eine besonders günstige Vergrößerung des Wirkungsspektrums wird mit folgenden Fungiziden erzielt:

Manganethylenbisdithiocarbamat
Mangan-Zinkethylenbisdithiocarbamat
Ammoniak-Komplex von Zink-(N,N-ethylen-bis-dithiocarbamat)
N-Trichlormethylthio-tetrahydrophthalimid
N-Trichlormethyl-phthalimid
5-Ethoxy-3-trichlor methyl-1,2,3-thiadiazol
2-Methoxycarbonylamino-benzimidazol
2-Rhodanmethylthiobenzthiazol
1,4-Dichlor-2,5-dimethoxybenzol
2,3-Dichlor-6-methyl-1,4-oxathiin-5-carbonsäureanilid
2-Methyl-5,6-dihydro-4-H-pyran-3-carbonsäure-anilid
2,4,5-Trimethyl-furan-3-carbonsäureanilid
2-Methyl-furan-3-carbonsäureanilid
2,5-Dimethyl-furan-3-carbonsäurecyclohexylamid
N-Cyclohexyl-N-methoxy-2,5-dimethyl-furan-3-carbonsäure-amid
5-Methyl-5-vinyl-3-(3,5-dichlorphenyl)-2,4-dioxo-1,3-oxa-zolidin
3-(3,5-Dichlorphenyl)-5-methyl-5-methoxymethyl-1,3-oxazolidin-2,4-dion

Die Verbindungen können aber auch mit folgenden Fungiziden kombiniert werden:

Schwefel,
Dithiocarbamate und deren Derivate, wie
Ferridimethyldithiocarbamat
Zinkdimethyldithiocarbamat
Zinkethylenbisdithiocarbamat
Tetramethylthiuramdisulfide
Zink-(N,N-propylen-bis-dithiocarbamat)
Ammoniak-Komplex von Zink-(N,N'-propylen-bis-dithiocarbamat) und
N,N'-Polypropylen-bis-(thiocarbamoyl)-disulfid
Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
h eterocyclische Strukturen, wie
2-Heptadecyl-2-imidazolin-acetat
2,4-Dichlor-6-(o-chloranilino)-s-triazin
0,0-Diethyl-phthalimidophosphonothioat
5-Amino-1-(bis-(dimethylamino)-phosphinyl)-3-phenyl-1,2,4-triazol)
2,3-Dicyano-1,4-dithiaanthrachinon
2-Thio-1,3-dithio-(4,5,-b)-chinoxalin
1-(Butylcarbamoyl)-2-benzimidazol-carbaminsäuremethyl-ester
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin-4,4-dioxid
2,3-Dihydro-5-carboxanilido-6-methyl-1,4-oxathiin
2-(Furyl-(2))-benzimidazol
Piperazin-1,4-di-yl-bis-(1-(2,2,2-trichlor-ethyl)-formamid
2-(Thiazolyl-(4)-benzimidazol
5-Butyl-2-dimethylamino-4-hydroxy-6-methyl-pyrimidin

Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol
sowie verschiedene Fungizide, wie
Dodecylguanidinacetat
3-(3-(3,5-Dimethyl-2-oxycyclohexyl)-2-hydroxyethyl)-glu-tarimid > >exachlorbenzol
N-Dichlorfluormethylthio-N',N'-dimethyl-N-phenyl-schwefelsäurediamid
2,5-Dimethyl-furan-3-carbonsäureanilid
2-Methyl-benzoesäure-anilid
2-Jod-benzoesäure-anilid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
alpha-(2-Chlorphenyl)-alpha-(4-chlorphenyl)-5-pyrimidin-methanol.
N-Formyl-N-morpholin-2,2,2-trichlorethylacetal
1-[2-(2,4-Dichlorphenyl)-4-ethyl-1,3-dioxolan-2-yl-methyl]-1H-1,2,4-triazol
1-[2-(2,4-Dichlorphenyl)-4-n-propyl-1,3-dioxolan-2-yl-methyl]-1H-1,2,4-triazol
DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
5-Nitro-isophthalsäure-di-isopropylester,
N-(2,6-Dimethylphenyl)-N-chloracetyl-D,L-2-aminobutyro-lacton,
N-(n-Propyl)-N-(2,4,6-trichlorphenoxyethyl)-N'-imidazolyl-harnstoff,
N-[3-(p-tert.-Butylphenyl)-2-methyl-propyl]-cis-2,6-di-methylmorpholin.

Die Wirkstoffe werden beispielsweise in Form von direkt versprühbaren Lösungen, Pulvern, Suspensionen, auch hochprozentige wäßrige, ölige oder sonstige Suspensionen oder Dispersionen, Emulsionen, Öldispersionen, Pasten, Stäubemitteln, Streumitteln, Granulaten, durch Versprühen, Vernebeln, Verstäuben, Verstreuen oder Gießen ausgebracht. Die Aufwendungsformen richten sich ganz nach den Verwendungszwecken; sie sollten in jedem Fall möglichst die feinste Verteilung der neuen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate z.B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z.B. Dimethylformamid, Dimethylsulfoxid, N-Methylpyrrolidon, Wasser in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergieroder Imulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäuren, Phenolsulfonsäure, Alkylarylsulfonate, Alkyl-Sulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole,

Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctylphenol-, Octylphenol-, Nonylphenol-, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Silicagel, Kieselsäuren, Kieselgele, Talkum, Bolus, Löß, Ton, Dolomit, Diatommeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehle, Baumrinden-, Holz- und Nußschalenmehl, Cellulosepulver und andere feste Trägerstoffe.

Beispiele für solche Zubereitungen sind:

I. Man vermischt 90 Gew.-Teile der Verbindung 3 mit 10 Gew.-Teilen N-Methyl-alpha-pyrrolidon und erhält

eine Lösung, die zur Anwendung in Form kleinster Tropfen geeignet ist.

II. 20 Gew.-Teile der Verbindung 38 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-mono-ethanolamin, 5 Gew.-Teilen Calciumsalz der Dodecylbenzolsulfonsäure und 5 Gew.-Teilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 26 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 38 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an .1 Mol Ricinusöl besteht. Durch Eingießen und feines Verteilen der Lösung in Wasser erhält man eine wäßrige Dispersion.

V. 80 Gew.-Teile der Verbindung Nr. 43 werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 5 Gew.-Teile der Verbindung Nr. 57 werden mit 95 Gew.-Teilen feinteiligem Kaolin innig vermischt. Man erhält auf diese Weise ein Stäubemittel, das 5 Gew.-% des Wirkstoffs enthält.

VII. 30 Gew.-Teile der Verbindung Nr. 58 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 59 werden mit 30 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion.

IX. 20 Teile der Verbindung Nr. 60 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

Die folgenden Versuche belegen die biologische Wirkung der neuen Verbindungen. Vergleichsmittel ist der bekannte Wirkstoff 5-Chlor-7-brom-8-hydroxychinolinacrylsäureester (A).

**Versuch 1**

Wirksamkeit gegen Botrytis cinerea an Paprika

Paprikasämlinge der Sorte "Neusiedler Ideal Elite" werden, nachdem sich 4 bis 5 Blätter gut entwickelt haben, mit wäßrigen Suspensionen, die 80 % Wirkstoff und 20 % Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22 bis 24°C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Das Ergebnis des Versuches zeigt, daß beispielsweise die Verbindungen Nr. 38, 43, 48, 57, 58, 59, 60, 106, 113 und 131 bei der Anwendung als 0,05 %ige Spritzbrühe eine bessere (beispielsweise 90 %) fungizide Wirkung haben als das bekannte Mittel A (beispielsweise 50 %).

**Versuch 2**

Wirksamkeit gegen Aspergillus niger

Zur Prüfung der Wirksamkeit werden die Wirkstoffe einer für das Wachstum des Pilzes Aspergillus niger optimalen Nährlösung in Mengen von 100, 50, 25, 10, 5 und 1 Gew.-Teilen pro Million Teile Nährlösung zugesetzt. Jeweils 10 ml Nährlösungs-Wirkstoffgemisch werden in sterile Reagenzgläser gegeben und mit einem Tropfen einer Sporensuspension beimpft, die $10^6$ Konidien enthält. Nach 12O-ständiger Bebrütung bei 37°C werden aus denjenigen Röhrchen, die kein sichtbares Pilzwachstum zeigen, Proben entnommen und auf Pilznährböden übertragen. In der Tabelle wird die Verdünnungsstufe angegeben, bei welcher nach dem Übertragen einer Probe auf den Nährboden kein Wachstum des Pilzes mehr erfolgt.

Das Ergebnis des Versuches zeigt, daß beispielsweise die Verbindungen 26, 38, 43, 57 und 58 (beispielsweise bei Verdünnung von 10 Teilen Wirkstoff pro Million Teile Lösung) eine gute fungizide Wirkung haben.

**Patentansprüche** für die Vertragsstaten BE, CH, DE, FR, GB,

IT, LI, NL, SE

1. Substituiertes Chinolinderivat der Formel I

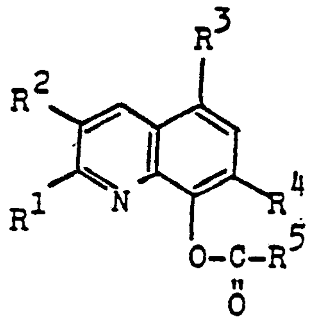

I

in der
$R^1$ Wasserstoff oder Methyl,
$R^2$ Wasserstoff oder Halogen,
$R^3$ Wasserstoff, Acetyl, Halogen oder Nitro,
$R^4$ Wasserstoff, Halogen oder Nitro und
$R^5$ einen gegebenenfalls durch einen $C_1$-$C_4$-Alkylrest, einen $C_1$-$C_4$-Halogenalkylrest, eine Nitrogruppe oder ein Halogenatom einfach oder unabhängig voneinander zweifach substituierten Thiophen-, Pyrrol-, Oxazol-, Thiazol-, Imidazol-, Isoxazol-, Isothiazol-, Pyrazol-, 1,3,4-Thiadiazol-, 1,3,4-Oxadiazol-, 1,2,4-Thiadiazol-, 1,2,4-Oxadiazol-, 1,2,4-Triazol-, 1,2,3-Thiadiazol-, 1,2,3-Oxadiazol-, 1,2,3-Triazol-, 1,2,5-Thiadiazol- oder 1,2,5-Oxadiazol-Rest, oder einen halogen- oder mono- oder dimethylsubstituierten Furan-Rest bedeutet.

2. Chinolinderivat der Formel I gemäß Anspruch 1, in der $R^1$ Wasserstoff, $R^2$ Wasserstoff oder Halogen, $R^3$ Acetyl, Halogen oder Nitro, $R^4$ Halogen oder Nitro und $R^5$ einen gegebenenfalls durch Chlormethyl substituierten Isoxazol-(3)- oder -(5)-Rest, einen gegebenenfalls durch Methyl oder Chlor substituierten Thiophen-(2)-, -(3)- oder -(5)-Rest, einen gegebenenfalls durch Methyl substituierten Oxazol-(5)-Rest, oder einen gegebenenfalls durch Methyl substituierten 1,2,3-Thiadiazol-(4)- oder -(5)-Rest bedeutet.

3. Verfahren zur Herstellung von substituierten Chinolin-derivaten der Formel I gemäß Anspruch 1, dadurch gekennzeichnet, daß man eine Verbindung der Formel II

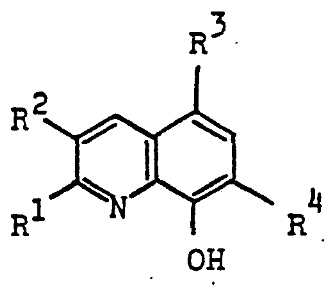

II

in der
$R^1$, $R^2$, $R^3$ und $R^4$ die in Ancpruch 1 angegebene Bedeutung haben oder ihre Alkali- oder Erdalkalisalze mit einem Carbonsäurederivat der Formel III

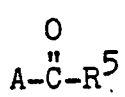

III

in der
$R^5$ die in Anspruch 1 genannte Bedeutung hat und A für eine nucleophil verdrängbare Abgangsgruppe steht, umsetzt.

4. Mikrobizides Mittel, enthaltend ein substituiertes Chinolinderivat der Formel I gemäß Anspruch 1.

5. Verfahren zur Bekämpfung von Pilzen, dadurch gekennzeichnet, daß man ein substituiertes Chinolinderivat der Formel I gemäß Anspruch 1 auf die Pilze oder auf durch Pilzbefall bedrohte Flächen, Materialien, Pflanzen oder Saatgüter einwirken läßt.

**Patentansprüche** für den Vertragsstaat AT

1. Mikrobizid, enthaltend ein substituiertes Chinolinderivat der Formel I

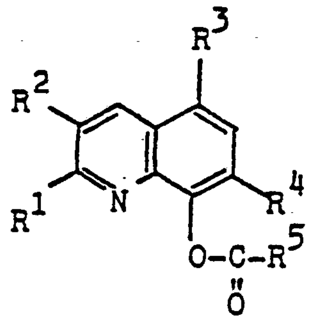

I

in der
$R^1$ Wasserstoff oder Methyl,
$R^2$ Wasserstoff oder Halogen,
$R^3$ Wasserstoff, Acetyl, Halogen oder Nitro,
$R^4$ Wasserstoff, Halogen oder Nitro und
$R^5$ einen gegebenenfalls durch einen $C_1$-$C_4$-Alkylrest, einen $C_1$-$C_4$-Halogenalkylrest, eine Nitrogruppe oder ein Halogenatom einfach oder unabhängig voneinander zweifach substituierten Thiophen-, Pyrrol-, Oxazol-, Thiazol-, Imidazol-, Isoxazol-, Isothiazol-, Pyrazol-, 1,3,4-Thiadiazol-, 1,3,4-Oxadiazol-, 1,2,4-Thiadiazol-, 1,2,4-Oxadiazol-, 1,2,4-Triazol-, 1,2,3-Thiadiazol-, 1,2,3-Oxadiazol-, 1,2,3-Triazol-, 1,2,5-Thiadiazol- oder 1,2,5-Oxadiazol-Rest, oder einen halogen- oder mono- oder dimethylsubstituierten Furan-Rest bedeutet.

2. Mikrobizid der Formel I gemäß Anspruch 1, dadurch gekennzeichnet, daß $R^1$ Wasserstoff, $R^2$ Wasserstoff oder Halogen, $R^3$ Acetyl, Halogen oder Nitro, $R^4$ Halogen oder Nitro und $R^5$ einen gegebenenfalls durch Chlormethyl substituierten Isoxazol-(3)- oder -(5)-Rest, einen gegebenenfalls durch Methyl oder Chlor substituierten Thiophen-(2)-, -(3)- oder -(5)-Rest, einen gegebenenfalls durch Methyl substituierten Oxazol-(5)-Rest, oder einen gegebenenfalls durch Methyl substituierten 1,2,3-Thiadiazol-(4)- oder -(5)-Rest bedeutet.

3. Verfahren zur Herstellung von substituierten Chinolin-derivaten der Formel I gemäß Anspruch 1, dadurch gekennzeichnet, daß man eine Verbindung der Formel II

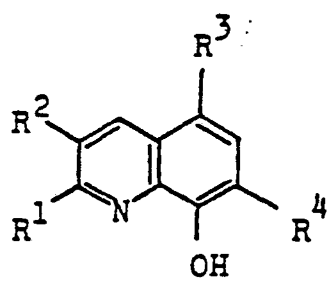

II

in der
$R^1$, $R^2$, $R^3$ und $R^4$ die in Anspruch 1 angegebene Bedeutung haben oder ihre Alkali- oder Erdalkalisalze mit einem Carbonsäurederivat der Formel III

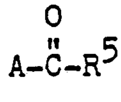

III

in der
$R^5$ die in Anspruch 1 genannte Bedeutung hat und A für eine nucleophil verdrängbare Abgangsgruppe steht, umsetzt.

4. Mikrobizid gemäß Anspruch 1, enthaltend ein substituiertes Chinolinderivat der Formel I gemäß Anspruch 1 und einen inerten Trägerstoff.

5. Verfahren zur Bekämpfung von Pilzen, dadurch gekennzeichnet, daß man ein substituiertes Chinolinderivat der Formel I gemäß Anspruch 1 auf die Pilze oder auf durch Pilzbefall bedrohte Flächen, Materialien, Pflanzen oder Saatgüter einwirken läßt.

**Claims** for the Contracting states: Be, CVh, De, FR, GB,

IT, LI, NL, SE

1- A substituted quinoline derivative of the formula

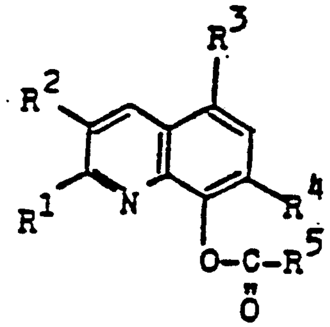

I,

where

$R^1$ is hydrogen or methyl,

$R^2$ is hydrogen or halogen,

$R^3$ is hydrogen, acetyl, halogen or nitro,

$R^4$ is hydrogen, halogen or nitro, and

$R^5$ is thienyl, pyrryl, oxazolyl, thiazolyl, imidazolyl, isoxazolyl, isothiazolyl, pyrazolyl, 1,3,4-thiadiazolyl, 1,3,4-oxadiazolyl, 1,2,4-thiadiazolyl, 1,2,4-oxadiazolyl, 1,2,4-triazolyl, 1,2,3-thiadiazolyl, 1,2,3-oxadiazolyl, 1,2,3-triazolyl, 1,2,5-thiadiazolyl or 1,2,5-oxadiazolyl optionally mono- or disubstituted by alkyl of 1 to 4 carbon atoms, haloalkyl of 1 to 4 carbon atoms, nitro or halogen, or is furyl which is halogen-substituted or monomethyl- or dimethyl-substituted.

2. A quinoline derivative of the formula I as claimed in claim 1, where $R^1$ is hydrogen, $R^2$ is hydrogen or halogen, $R^3$ is acetyl, halogen or nitro, $R^4$ is halogen or nitro, and $R^5$ is optionally chloromethyl-substituted isoxazol-3-yl or isoxazol-5-yl, optionally methyl- or chlorosubstituted thien-2-yl, thien-3-yl or thien-5-yl, optionally methyl-substituted oxazol-5-yl, or optionally methyl-substituted 1,2,3-thiadiazol-4-yl or 1,2,3-thiadiazol-5-yl.

3. A process for the preparation of a substituted quinoline derivative of the formula I as claimed in claim 1, wherein a compound of the formula II

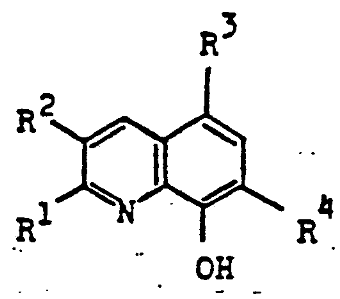

II,

where

$R^1$, $R^2$, $R^3$ and $R^4$ have the meanings given in claim 1, or an alkali metal or alkaline earth metal salt thereof is reacted with a carboxylic acid derivative of the formula III

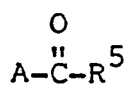

III,

where

$R^5$ has the meanings given in claim 1, and A denotes a nucleophilically displaceable leaving group.

4. A microbicide containing a substituted quinoline derivative of the formula I as claimed in claim 1.

5. A method of combating fungi, wherein a substituted quinoline derivative of the formula I as claimed in claim 1 is allowed to act on the fungi or the areas, materials, plants or seed threatened by fungal attack.

**Claims** for the Contracting state: AT

1. A microbicide containing a substituted quinoline derivative of the formula

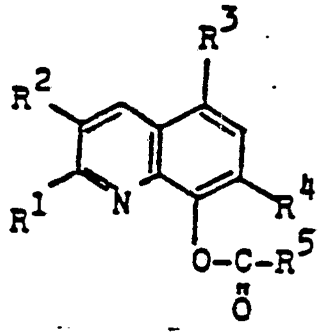

I,

where
$R^1$ is hydrogen or methyl,
$R^2$ is hydrogen or halogen,
$R^3$ is hydrogen, acetyl, halogen or nitro,
$R^4$ is hydrogen, halogen or nitro, and
$R^5$ is thienyl, pyrryl, oxazolyl, thiazolyl, imidazolyl, isoxazolyl, isothiazolyl, pyrazolyl, 1,3,4-thiadiazolyl, 1,3,4-oxadiazolyl, 1,2,4-thiadiazolyl, 1,2,4-oxadiazolyl, 1,2,4-triazolyl, 1,2,3-thiadiazolyl, 1,2,3-oxadiazolyl, 1,2,3-triazolyl, 1,2,5-thiadiazolyl or 1,2,5-oxadiazolyl optionally mono- or disubstituted by alkyl of 1 to 4 carbon atoms, haloalkyl of 1 to 4 carbon atoms, nitro or halogen, or is furyl which is halogen-substituted or monomethyl- or dimethyl-substituted.

2. A microbicide of the formula I as claimed in claim 1, where $R^1$ is hydrogen, $R^2$ is hydrogen or halogen, $R^3$ is acetyl, halogen or nitro, $R^4$ is halogen or nitro, and $R^5$ is optionally chloromethyl-substituted isoxazol-3-yl or isoxazol-5-yl, optionally methyl- or chloro-substituted thien-2-yl, thien-3-yl or thien-5-yl, optionally methylsubstituted oxazol-5-yl, or optionally methyl-substituted 1,2,3-thiadiazol-4-yl or 1,2,3-thiadiazol-5-yl.

3. A process for the preparation of a substituted quinoline derivative of the formula I as claimed in claim 1, wherein a compound of the formula II

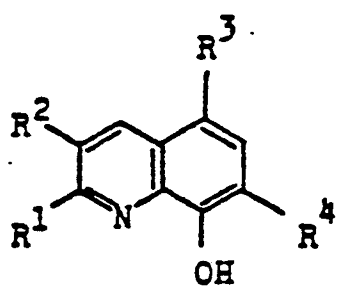

II,

where
$R^1$, $R^2$, $R^3$ and $R^4$ have the meanings given in claim 1, or an alkali metal or alkaline earth metal salt thereof is reacted with a carboxylic acid derivative of the formula III

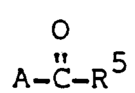

III,

where
$R^5$ has the meanings given in claim 1, and A denotes a nucleophilically displaceable leaving group.

4. A microbicide as claimed in claim 1, containing a substituted quinoline derivative of the formula I as claimed in claim 1 and an inert carrier.

5. A method of combating fungi, wherein a substituted quinoline derivative of the formula I as claimed in claim 1 is allowed to act on the fungi or the areas, materials, plants or seed threatened by fungal attack.

**Revendications** pour les Etats contractants: BE, CH, DE, FR, GB,

IT, LI, NL, SE

1.- Dérivé substitué de quinoléine de formule I

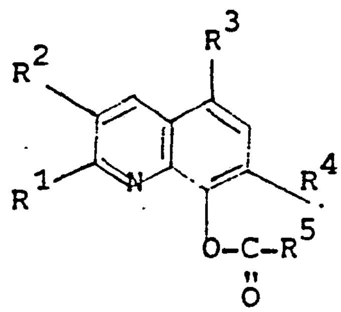

I

dans laquelle
$R^1$ représente hydrogène ou méthyle,
$R^2$ hydrogène ou halogène
$R^3$ hydrogène, acétyle, halogène ou nitro,
$R^4$ hydrogène, halogène ou nitro et
$R^5$ un reste thiophène, pyrrole, oxazole, thiazole, imidazole, isoxazole, isothiazole, pyrazole, 1,3,4-thiadiazole, 1,3,4-oxadiazole, 1,2,4-thiadiazole, 1,2,4-oxadiazole, 1,2,4-triazole, 1,2,3-thiadiazole, 1,2,3-oxadiazole, 1,2,3-triazole, 1,2,5-thiadiazole ou 1,2,5-oxadiazole, éventuellement substitué une fois ou, indépendant l'un de l'autre, deux fois, par un reste alkyle en $C_1$-$C_4$, un reste halogénalkyle en $C_1$-$C_4$, un groupe nitro ou un atome d'halogène, ou un reste furane substitué par un halogène ou par mono- ou diméthyle.

2.- Dérivé de quinoléine de formule I, selon la revendication 1, dans lequel $R^1$ représente hydrogène, $R^2$ hydrogène ou halogène, $R^3$ acétyle, halogène ou nitro, $R^4$ halogène ou nitro et $R^5$ un reste isoxazole-(3)- ou -(5)- éventuellement substitué par chlorométhyle, un reste thiophène-(2)-, -(3)- ou -(5)-, éventuellement substitué par méthyle ou chlore, un reste oxazole -(5)- éventuellement substitué par méthyle, ou un reste 1,2,3-thiadiazole -(4)- ou -(5)-, éventuellement substitué par méthyle.

3.- Procédé de préparation de dérivés substitués de quinoléine de formule I selon la revendication 1, caractérisé par le fait que l'on fait réagir un composé de formule II

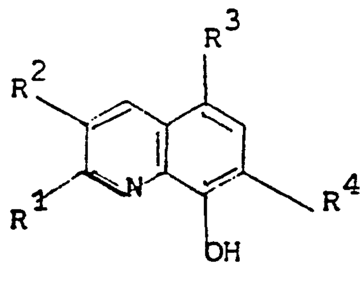

II

dans laquelle
$R^1$, $R^2$, $R^3$ et $R^4$ ont significations données dans la revendication 1, ou son sel alcalin ou alcalinoterreux, avec un dérivé d'acide carboxylique de formule III

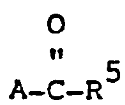

III

dans laquelle
$R^5$ a la signification indiquée dans la revendication 1 et A représente un groupe éliminable, déplaçable par action nucléophile.

4.- Agent microbicide, contenant un dérivé substitué de quinoléine de formule I selon la revendication 1.

5.- Procédé de lutte contre les champignons, caractérisé par le fait que l'on fait agir un dérive substitué de quinoléine de formule I, selon la revendication 1, sur les champignons ou les surfaces, matériaux, plantes ou semences menacés d'une attaque par des champignons.

**Revendications** pour l'Etat contractant: AT

1.- Microbicide contenant un dérivé substitué de quinoléine de formule I

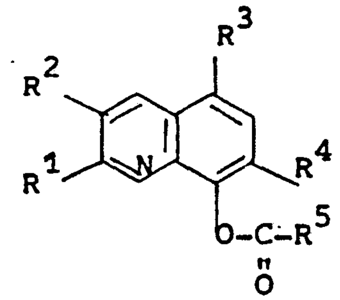

I

dans laquelle
$R^1$ représente hydrogène ou méthyle,
$R^2$ hydrogène ou halogène,
$R^3$ hydrogène, acétyle, halogène ou nitro,
$R^4$ hydrogène, halogène ou nitro et
$R^5$ un reste thiophène, pyrrole, oxazole, thiazole, imidazole, isoxazole, isothiazole, pyrazole, 1,3,4-thiadiazole, 1,3,4-oxadiazole, 1,2,4-thiadiazole, 1,2,4-oxadiazole, 1,2,4-triazole, 1,2,3-thiadiazole, 1,2,3-oxadiazole, 1,2,3-triazole, 1,2,5-thiadiazole ou 1,2,5-oxadiazole, eventuellement substitué une fois ou, indépendant l'un de l'autre, deux fois, par un reste alkyle en $C_1$-$C_4$, un reste halogénalkyle en $C_1$-$C_4$, un groupe nitro ou un atome d'halogène, ou un reste furane substitué par un halogène ou par mono- ou diméthyle.

2.- Microbicide de formule I, selon la revendication 1, caractérisé par le fait que $R^1$ représente hydrogène, $R^2$ hydrogène ou halogène, $R^3$ acétyle, halogène ou nitro, $R^4$ halogène ou nitro et $R^5$ un reste isoxazole-(3)- ou -(5)- éventuellement substitué par chlorométhyle, un reste thiophène-(2)-, -(3)- ou -(5), éventuellement substitué par méthyle ou chlore, un reste oxazole-(5)- éventuellement substitué par méthyle, ou un reste 1,2,3-thiadiazole-(4)- ou -(5)-, éventuellement substitué par méthyle.

3.- Procédé de préparation de dérivés substitués de quinoléine de formule I selon la revendication 1, caractérisé par le fait que l'on fait réagir un composé de formule II

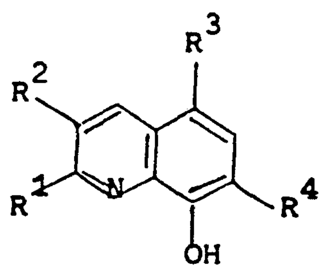

dans laquelle
$R^1$, $R^2$, $R^3$ et $R^4$ ont les significations données dans la revendication 1, ou son sel alcalin ou alcalinoterreux, avec un dérivé d'acide carboxylique de formule III

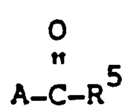

III

dans laquelle $R^5$ a la signification indiquée dans la revendication 1 et A représente un groupe éliminable, déplaçable par action nucléophile.

4.- Microbicide selon la revendication 1, contenant un dérivé substitué de quinoléine de formule I selon la revendication 1, et un support inerte.

5.- Procédé de lutte contre les champignons, caractérisé par le fait que l'on fait agir un dérivé substitué de quinoléine de formule I selon la revendication 1, sur les champignons ou les surfaces, matériaux, plantes ou semences menacés d'une attaque par des champignons.